# EUROPEAN PATENT APPLICATION

(11) **EP 1 859 737 A1**
(43) Date of publication of application: **28.11.2007**
(21) Application number: 07290632.4
(22) Date of filing: 16.05.2007
(51) Int. Cl.: A61B 5/0488

(54) **Living body information measuring apparatus and system**

(30) Priority: 24.05.2006 JP 2006144388
(71) Applicant: CASIO COMPUTER CO., LTD., Shibuya-ku, Tokyo 151-8543 (JP)
(72) Inventor: Fukumura, Masaaki, c/o Casio Computer Co., Ltd., Hamura-shi 205-8555 Tokyo (JP); Kanzaki, Takashi, c/o Casio Computer Co., Ltd., Hamura-shi 205-8555 Tokyo (JP)
(74) Representative: Bertrand, Didier

(57) **Abstract**

A myoelectric potential produced across a predetermined muscle of a living body in isometric exercise is measured before physical training. An item of myoelectric potential information involving the measured myoelectric potential is stored as a reference recovery value, which is then compared with an item of myoelectric potential information involving a myoelectric potential measured after the training, thereby determining an extent of recovery of the muscle.

## Description

The present invention relates to living body information measuring apparatus and systems, and more particularly to such apparatus and system that measure a myoelectric potential which will be produced across a predetermined muscle of a living body.

Living body measuring systems have been known hitherto that measure changes in living body information such as myoelectric potentials, pulses, blood pressures and temperatures of the living body. The systems are widely used not only as medical equipment, but also for maintaining a user's health and grasping the state of his or her training.

For example, Unexamined Japanese Patent Publication 2004-202196 discloses a body information measuring system that measures a myoelectrical potential produced across a part of a user's body on which the system is worn, estimates the state of muscles of the part and displays results of the estimation. Unexamined Japanese Patent Publication 60-168435 discloses a measuring device that compares respective myoelectric potentials of a user produced in his or her muscular activities with a maximum myoelectric potential value recorded, thereby calculating a maximum muscular strength ratio in real time so as to allow the extent of a load imposed on the muscles to be evaluated in real time. Unexamined Japanese Patent Publication 2000-232 discloses a muscle fatigue determining apparatus that compares myoelectric potentials produced across a user's muscles before and after his or her training, and then determines an extent of the muscular fatigue. These publications disclose that the user can take physical training or training while confirming his or her muscular state.

When the user takes physical training, which is hereinafter represented simply as physical training, which includes moving his or her muscles for strengthening or rehabilitating purposes it is important for him or her to plan the training after knowing his or her muscular fatigue and recovery state. Those publications disclose determining to how extent the muscles are fatigued at the time of its measurement, but fail to disclose knowing when the muscles have been recovered.

For example, when the user takes physical training, fine cracks will occur in the muscular fibers and the muscles will be broken due to various causes such as exhaustion of muscular glycogen and accumulation of lactic acid. Thus, the total amount of muscles will temporarily decrease compared to that present before the training, and the functions of the muscles will be reduced temporality. Then, when the user reposes appropriately, his or her muscles will be restored and recovered gradually. After a predetermined time, muscular fibers thicker than before the training will be formed and the total amount of muscles will increase. Such recovered muscular state is called "superrecovery".

Conversely, if next physical training is taken before the muscular fibers are sufficiently recovered after cracked and broken by the training, cracks and breakage of the muscular fivers would further advance and hence a long time would be required for complete recovery and restoration of the muscles. If the user continues to get unreasonable training in such state, the total amount of his or her muscles would decrease gradually. If the muscles are left for more than a certain time after the "superrecovery" is reached, the total amount of the muscles would decrease gradually, which would not produce a sufficient effect of the training.

In order to take physical (more particularly muscular) training effectively, it is preferable that a sufficient recovery time or rest should be ensured before the "superrecovery" is reached after the training and then that next training should be started. Continuation of the raining in this manner would increase the total amount of muscles and the muscular strength effectively.

To this end, it is required to grasp a time when the "superrecovery" state is reached, and then appropriately determine a time when next training starts. The above-mentioned patent publications only disclose a method of knowing the state of the muscles such as how the muscles are fatigued at the measurement and fails to disclose a method of knowing when the muscles have been recovered or whether or not the muscles are in the "superrecovery" state.

It is said that a recovery period of approximately 24-72 hours is required for the muscles to reach the "superrecovery" state after the training. The recovery period varies depending on various factors such as the extent of each user's training, the part of his or her body concerned, how long he or she took a rest and how well he or she is nourished. Thus, in the past, whether or not the user's muscles are in the "superrecovery" state must be determined by his or her sensations. Thus, it is impossible to accurately determine when next physical training should be taken after the muscles are fatigued due to the previous training.

It is therefore an object of the present invention to provide a living body information measuring apparatus and system capable of determining whether a user's muscle is in the "superrecovery" state.

In accordance with the living body information measuring apparatus and system of the present invention, myoelectric potentials produced across a predetermined muscle of a user's living body in isometric exercise before and after physical training are measured. The physical training involves, for example, cycling, walking, jogging, marathon or weight training. An item of myoelectric potential information involving the myoelectric potential measured before the physical training is stored as a reference recovery value, which is then compared with the value of an item of myoelectric potential involving a myoelectric potential across the predetermined muscle measured after the training to determine an extent of recovery of the muscle.

The recovering speed of a user's muscle is different from that of another user's and from another of the same user. According to the apparatus, the reference recovery value acquired before the training is compared with the myoelectric potential information acquired after the training, thereby determining a degree of the muscle recovery. Thus, the time when the muscle has entered the "superrecovery" state is grasped without relying on the user's sensations and hence the user is allowed to take appropriate training.

The item of myoelectric potential information comprises at least one of an averaged rectified value, a root mean square value and an integrated value, as estimated indexes, for the amplitude of a waveform involving the measured myoelectric potential within a predetermined measurement period of time. The item of myoelectric potential information also may comprise at least one of a mean power frequency and a median frequency as estimated indexes for the frequency of the measured myoelectric potential waveform. The item of myoelectric potential information also may comprise a correlation coefficient between the estimated indexes for the amplitude and frequency in the predetermined measurement period of time.

When the value of the item of myoelectric potential involving the myoelectric potential measured after the training is larger than the reference recovery value, it is determined that the predetermined muscle has been recovered.

The predetermined measurement period of interval is arranged so as to start a selected time after measurement of the myoelectric potential in view of the fact that the myoelectric potential measured when the muscle was fatigued starts to change some time after the start of the measurement and the time when the myoelectric potential measured starts to change varies from user to user.

The start and end points of the predetermined measurement period of time may be set manually, for example, depending on a characteristic of advent of the user's fatigue, thereby allowing efficient measurement of the myoelectric potential.

Storage stores data on a recovery period of time between the end of the training and a time when the value of the item of myoelectric potential information involving the myoelectric potential measured after the training has exceeded the reference recovery value. When a time corresponding to the recovery period of time has elapsed from the end of next training, the user is informed of this fact. Thus, the user can appropriately determine when measurement should be made to confirm the recovered state of the muscle.

The above and other objects, aspects and features of the present invention will now become apparent to those of ordinary skill in the art upon review of the following description of a specific embodiment of the present invention in conjunction with the accompanying drawings in which:
FIG. 1 illustrates a living body information measuring system worn on a left arm of a user;
FIG. 2 is a block diagram of two myoelectric potential measuring devices and a controller that compose the living body information measuring system;
FIGS. 3A and 3B each show selection of a different point in time as a measurement start point;
FIGS. 4A, 4B and 4C show ARV, MPF and γ ARV-MPF characteristics, respectively, of myoelectric potential information, wherein solid and broken lines represent those characteristics obtained when the muscles are fatigued and recovered from the fatigue, respectively;
FIG. 5 shows a configuration of storage of the myoelectric measuring device;
FIGS. 6A and 6B show a muscular "superrecovery" state and a non-superrecovery state, respectively;
FIG. 7 illustrates a specified configuration of storage of the controller;
FIG. 8 is a flowchart of processing to be performed by the myoelectric measuring device and the controller; and
FIG. 9 shows a flowchart to be continued to FIG. 8.

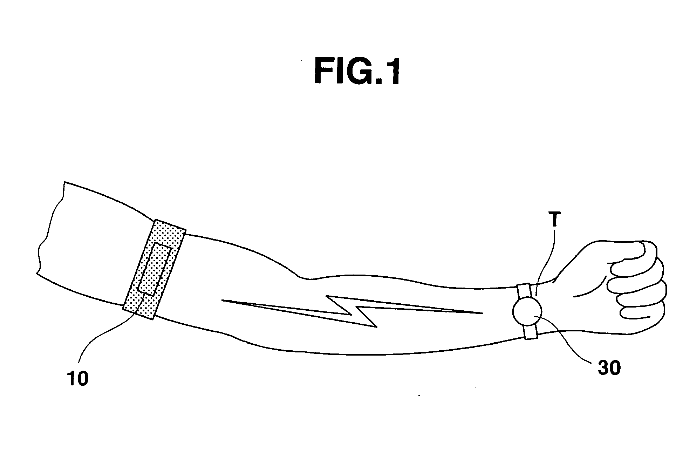

Referring to FIGS. 1-9, the living body information measuring system of one embodiment according to the present invention is capable of measuring at least myoelectric potentials produced as living body information in isometric exercise and physical training such as cycling training. The isometric exercise is, for example, a static one in which a user puts his or her certain strength into his or her muscles in the same posture without changing the joint angles of his or her arms, thereby increasing the tensile of the muscles without changing their overall length, one in which the user pushes with his or her hands against an unmovable object such as a wall or one in which the user strains his or her muscles intentionally without changing his or her posture. As an example, the following handles the living body information measuring system that measures a myoelectric potential produced across the user's muscles in isometric exercise in which the user intentionally strains his or her arm muscles.

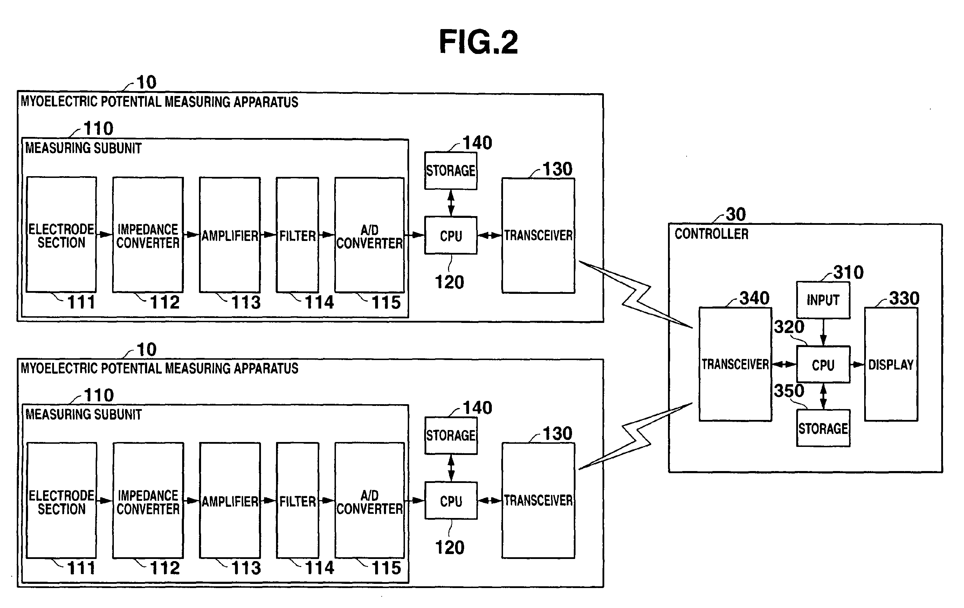

Referring to FIG. 1, the living body information measuring system 1 comprises a myoelectric measuring device 10 worn, for example, on the user's upper left arm and a wristwatch type controller 30 worn, for example, on the user's left wrist such that data communication is possible between device 10 and controller 30. If a myoelectric potential produced across the biceps of the user's upper arm when the biceps are intentionally strained without changing the user's posture as the isometric exercise is measured, measuring device 10 is worn on the user's upper arm at the position of the biceps on which an electrode section 111 (FIG. 2) is placed, for example, with a band. The user then operates controller 30 to start measurement of the myoelectric potential. Then, the user starts an - isometric exercise that includes putting his or her strength into his or her arms, without changing the joint angles of his or her arms, thereby straining the biceps of his or her upper arm. Controller 30 may be worn on the wrist of the same arm of the user as electrode section 111 is pasted on or on the wrist of the other arm of the user.

Referring to FIG. 2, the internal configuration and interconnection relationship of myoelectric measuring device 10 and controller 30 is shown. Device 10 comprises a measuring subunit 110, a CPU 120, a transceiver 130 and storage 140.

Measuring subunit 110 comprises electrode section 111 which includes a pair of electrodes (not shown) placeable in contact with a part of the user's body such as the biceps of his or her upper arm to sense a myoelectric potential produced across that part between the pair of electrodes, an impedance converter 112 that picks up, in a stabilized manner via a high input impedance circuit thereof, a potential sensed by electrode section 111, an amplifier 113 that amplifies a picked-up potential signal from converter 112 to a predetermined level, a filter 114 that allows only a predetermined frequency range of the signal from amplifier 113 to pass therethrough, and an A/D converter 115 that A/D converts a signal of that range from filter 114.

More specifically, changes in the potential detected by electrode section 111 are scores of microvolts (µ V) - several millivolts (mV) and the myoelectric potential waveform is in a frequency band of 2-10 KHz. As will be known from this fact, the living body impedance is very high and the sensed myoelectric potential is picked up and outputted in a stabilized manner by impedance converter (for example, including a voltage follower) 112. Then, amplifier (for example, including an operational amplifier) 113 approximately 100 times amplifies an output from converter 112 for processing the myoelectric potential waveform. The signal waveform amplified by amplifier 113 includes various superimposed noise. Thus, filter 114 eliminates frequency components other than the required frequencies of the myoelectric potential waveform. An analog output from filter 114 is digitized by A/D converter 115 (for example, of 12 bits).

While filter 114 is illustrated as being of an analog type, it may be of a digital type. In this case, the digital filter is required to be provided after A/D converter 115.

CPU 120 gives commands and transfers data to the respective functional elements of myoelectric potential measuring device 10 based on programs/data stored in storage 140 and data received from controller 30, and also controls measuring device 10. In the embodiment, CPU 120 performs a muscular state analysis process that includes analyzing the activity of the muscles based on the myoelectric potential (or living body information) measured continuously by measuring unit 110, and sending results of the analysis or measurement to controller 30.

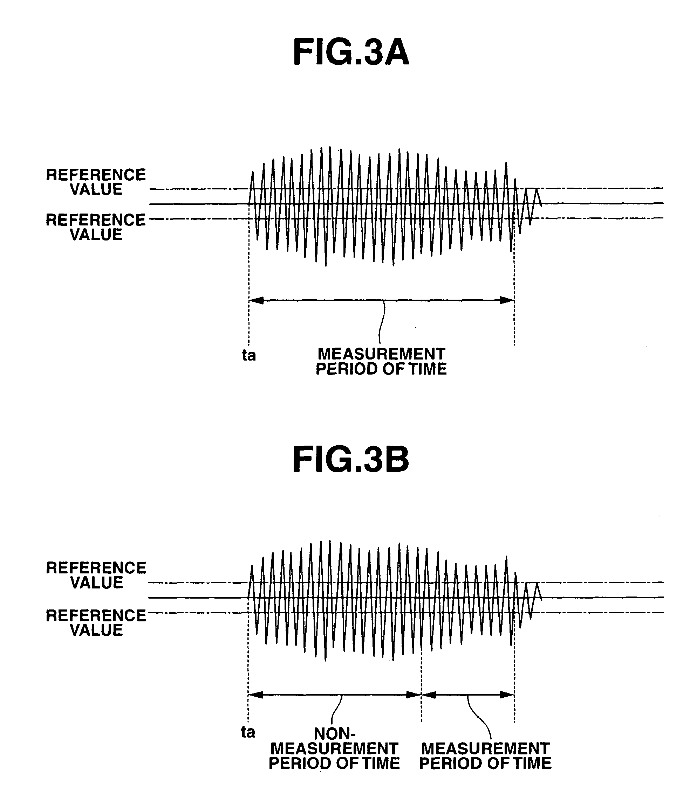

Referring to FIGS. 3A and 3B, this analysis process will be described more specifically. FIGS. 3A and 3B each show the waveform of a myoelectric potential sensed by measuring unit 110 worn on the biceps of the user's upper arm. The real muscular state analysis process is performed by causing CPU 120 to calculate an absolute value of a signal digitized by A/D converter 115. For convenience of explanation, this analysis will be described, using analog myoelectric potential waveforms of FIGS. 3A and 3B.

In this process, CPU 120 detects a time, ta, when the muscular activity starts, based on the measured myoelectric potential waveform and then analyzes the muscular state in the isometric exercise. More particularly, CPU 120 determines a time when the measured myoelectric potential exceeds a predetermined threshold at the muscular activity start point, ta. For example, as shown in.FIG. 3A, CPU 120 stores as produced myoelectric potential data 142 in storage 140 changes in the myoelectric potential with time in a measurement period of time starting at the muscle activity start point.

In order to grasp the muscular state in the isometric exercise appropriately, 20-30 seconds are appropriate as the period of time for measuring the myoelectric potential. However, the measuring period of time is not limited to this particular one and its measurement start point is not limited to this particular one either.

For example, as shown in FIG. 3B, the period setting may be such that the measurement start point is a predetermined time after the time, ta, when the muscular activity starts, that the time before the measurement start point composes a non-measurement period of time, and that a predetermined period of time after the measurement start point is the measurement period of time.

A measured value of the myoelectric potential increases abruptly irrespective of whether or not fatigue is present at the beginning of the isometric exercise. When the muscles are fatigued, the measured value of the myoelectric potential soon starts to lower. As just described, the fatigue of the muscles appears most remarkably in the isometric exercise some extent of time after the muscles are strained and the myoelectric potential has exceeded the predetermined threshold level. Thus, if a myoelectric potential is measured for a predetermined period of time starting a selected or predetermined time after the threshold is exceeded, the fatigue state of the muscles is grasped sufficiently.

The start and end points of the measurement period of time can be set at input unit 310. In this embodiment, a time when the myoelectric potential measured exceeds the threshold is set as a default start point of the measurement and a period of 30 seconds after the start point is set as a default measurement period of time. When no settings are made at input unit 310, the myoelectric potential is measured in the default measurement period of time.

From myoelectric potential data 142 indicative of changes in the myoelectric potential measured in the measurement period of time, CPU 120 calculates as myoelectric potential information an averaged rectified value (ARV), a root mean square value (RMS), and an integrated value, which are estimated indexes indicative of muscular tension and the amplitude of a waveform of myoelectric potentials obtained. CPU 120 also calculates from the myoelectric potential data 142 a mean power frequency (MPF) and a median frequency of the waveform indicative of fatigue of the muscles in the FTT analysis of the myoelectric potential waveform.

While in the embodiment CPU 120 is illustrated as calculating the above all as the myoelectric potential information, the myoelectric potential information which will be calculated by CPU 120 is not limited to that. For example, CPU 120 may calculate one or more items of the information. For example, when CPU 120 calculates as myoelectric potential information a correlation coefficient by dividing a covariance between any selected one of ARV, RMS and integrated value as the estimated indexes of the amplitude of the detected myoelectric potential waveform and any selected one of APF and MF as the estimated indexes of the frequency of the detected myoelectric potential waveform by both standard deviations of the selected estimated indexes.

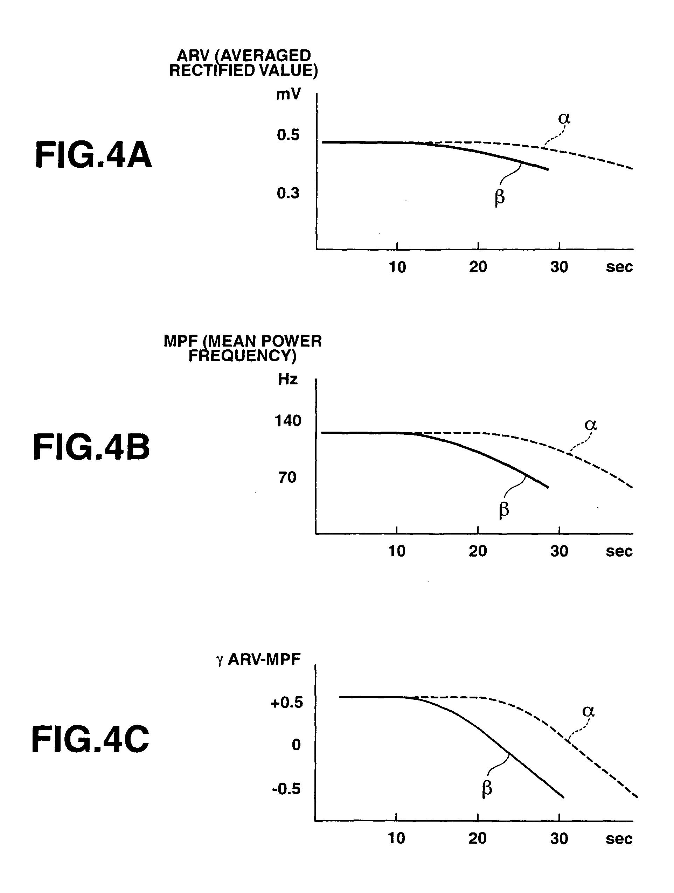

FIG. 4 illustrates one example of a graph indicative of changes in the myoelectric potential detected when the isometric exercise was taken for 30 seconds. FIGS. 4A and 4B show changes in ARV and MPF as estimated indexes for the amplitude and frequency, respectively, of the myoelectric potential waveform. FIG. 4C show changes in the correlation coefficient between ARV and MPF. Broken and solid curves α and β in each of FIGS.4A-4C show changes in the myoelectric potential data obtained when the muscles took a sufficient rest and were fatigued, respectively, before the physical training.

As shown in FIGS. 4A and 4B, even when any of ARV and MPF is used as the myoelectric potential information, its value starts to decrease gradually approximately 25 seconds after the start of the measurement when sufficient rest was taken before the physical training. In contrast, in the state where the muscles are fatigued after the physical training, the value of the ARV or MPF value starts to decrease gradually approximately 15 seconds after the start of the measurement. Since ARV and MPF are in a negative correlation, its correlation coefficient values are shown in broken and solid curves α and β of FIG. 4C obtained after the muscles took sufficient rest and are fatigued, respectively, as in FIGS. 4A and 4B, respectively.

As will be obvious from these FIGURES, the values of the myoelectric potential information obtained after the isometric exercise was taken when the muscles were fatigued will rapidly decrease compared to those obtained after the muscles took sufficient rest. Thus, the restored state of the muscles can be determined by checking changes in the values of the myoelectric potential information.

Transceiver 130 performs wireless communication with controller 30. For example, transceiver 130 demodulates a signal received from controller 30 via an antenna (not shown), sends a resulting signal to CPU 120, modulates and amplifies a control signal received from CPU 120 and then sends it via the antenna to controller 30.

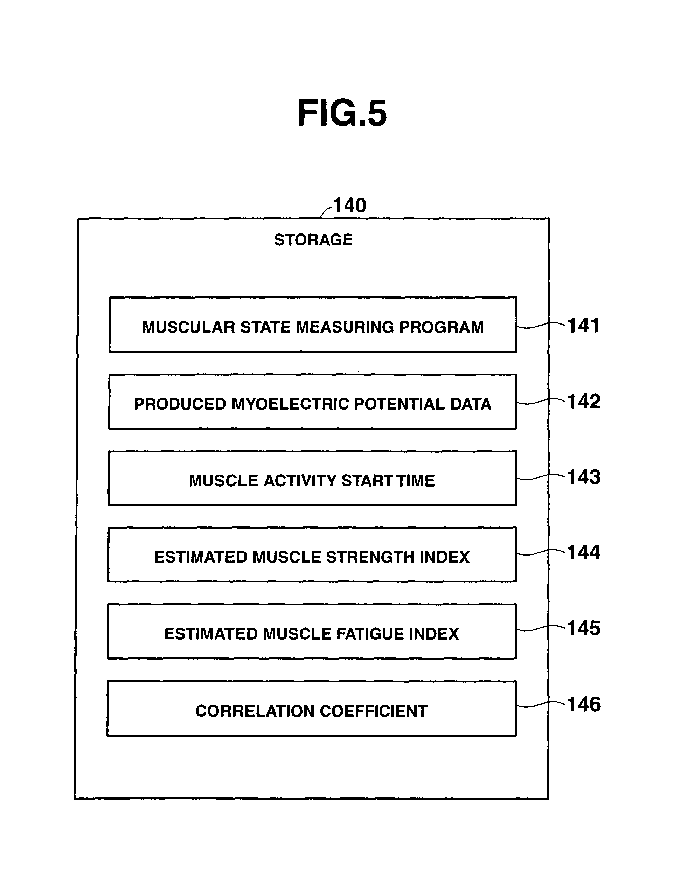

Storage 140 includes IC memories such as ROMs and/or RAMs, information storage media such as a hard disk and a reader that reads data from those memories and media. CPU 120 has stored a program to control myoelectric potential measuring device 10. FIG. 5 illustrates a specified configuration of storage 140 which has stored a muscular state analysis program 141 that executes the corresponding process. Storage 140 also has stored produced myoelectric potential data 142, muscular activity start time (ta) data 143, averaged rectified value (ARV) data 144, RMS data 145, integrated value data 146, MPF 147 and median frequency data 148 which are updated at each measurement.

As described above, CPU 120 calculates as myoelectric potential information a correlation coefficient by dividing a covariance between any selected one of ARV, RMS and integrated value as the estimated indexes for the amplitude of the detected myoelectric potential waveform and any selected one of APF and MF as the estimated indexes for the frequency of the detected myoelectric potential waveform by both standard deviations of the selected estimated indexes. Then, when the coefficient is to be used as the myoelectric potential information, CPU 120 stores the correlation coefficient in storage 140.

Referring to FIG. 2, controller 30 includes an input 310, CPU 320, display 330, transceiver 340 and storage 350.

Input unit 310 includes, for example, various switches, a dial and a touch panel. When operated, input unit 310 delivers to CPU 320 a corresponding signal indicative, for example, of an isometric exercise measurement start or end.

In this embodiment, input unit 310 functions to set the start and end times of a measurement period of time in which myoelectric potential measuring device 10 measures a myoelectric potential. A user can set at input unit 310 a measurement start point as a time corresponding to a desired potential above the threshold of the myoelectric potential, a measurement end time and an associated measurement period of time.

Display 330, which includes an LCD or ELD, displays results of measurement of the myoelectric potential by myoelectric potential measuring device 10 and the activity state of the user's muscles analyzed based on the results of the measurement. When CPU 320 determines that the restored state of the muscles are in a "superrestored" state to be described later in greater detail, display 330 displays this fact. When storage 350 has stored data on the restoration periods of time involving the measurements made in the past, and a restoration period of time has passed after the physical training, display 330 displays this fact under control of CPU 320, thereby functioning as means for informing the user about elapse of the restoration period of time.

CPU 320 gives commands and transfers data to the respective functional elements of controller 30 based on the programs and data stored in the storage 350, operation signals produced at input unit 310 and data received from myoelectric potential measuring device 10, thereby controlling controller 30 and the living body information measuring system 1. When a measurement start command is given at input unit 310, CPU 320 delivers a measurement start signal to myoelectric potential measuring device 10 and also determines a restored state of the muscles based on results of the analysis received at all times from myoelectric potential measuring device 10. On determining that the muscles are in the "superrecovery" state and that the predetermined recovery period of time has elapsed from the end of the physical training, CPU 320 causes display 330 to display that fact.

When receiving information on the myoelectric potential measured for the predetermined measurement period of time from measuring device 10 before the physical training, CPU 320 stores it as a reference recovery value in storage 350. Likewise, on receiving information indicative of the myoelectric potential measured for the predetermined measurement period of time from measuring device 10 after the physical training, CPU 320 stores it as new measured data in storage 350. On acquiring the reference recovery value and new measured data, CPU 320 reads out the muscle recovery state determining program from storage 350, compares the reference recovery value and the new measured data. When the new measured data exceeds the reference recovery value, or a value obtained by dividing the new measured data by the reference recovery value is larger than 1 (unity), CPU 320 determines that the muscles are in the "superrecovery" state. CPU 320 also calculates a time required from the end of the muscle training to the muscle "superrecovery" state, produces data on the muscular recovery period of time, and stores it in storage 350.

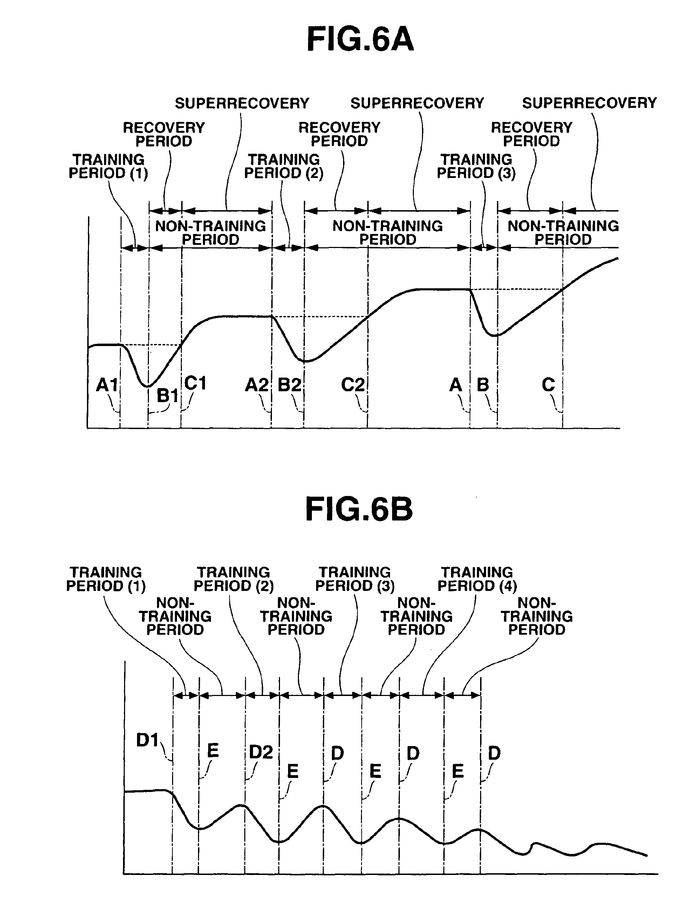

Referring to FIG. 6, the superrecovery of the muscles will be described. When the user takes physical training (such as moving his or her muscles for strengthening or rehabilitating purposes), the muscular fibers are then cracked and broken and the total amount of the muscles temporarily decreases compared to that before the training. Then, when no muscular training is taken for a certain interval of time or rest, the muscles are restored and recovered. After this, muscular fibers thicker than before the training are formed and the total amount of the muscles increases, which is called a "superrecovery" state.

More specifically, as shown in FIG. 6A, when physical training starts at a time of A1 after the user's muscles took a sufficient rest and then ends at an end B1 of a first training period of time (1), the muscular fibers are cracked and broken and the total amount of the muscles is decreased compared to that at the time of A1. Then, when no training is taken for a non-training interval of time, the muscles are gradually recovered. At a time of C1 the total amount of the muscles exceeds that at the time of A1 and then increases to a certain value (the interval of time from the time of B1 in FIG. 6A where the training ends to the time of C1 in FIG. 6A is referred to as "recovery interval of time").

A muscular state after the time of C1 in which the total amount of the muscles has increased compared to that at the time of A1 before the training is referred to as "superrecovery" state of the muscles. When another training or training is taken in a second training period of time (2) between times of A2 and B2 while the muscles are in the "superrecovery" state and then another non-training interval of time is provided, at a time of C2 the muscles enter the "superrecovery" state where the total amount of the muscles is larger than that before the training period of time (2). If the training period and non-training interval of time are provided repeatedly alternately, the total amount of the muscles increase gradually and the muscular strength is improved.

In contrast, as shown in FIG. 6B, if a next training period of time (2) starts at a time of D2 before a next "superrecovery" state would otherwise start in which the total amount of the muscles exceeds that at a time D1 of FIG. 6B, a further burden is imposed on the muscles before the muscles are not sufficiently recovered, and a long time is required for restoring and recovering the muscles. If unnatural training continues in such state, the total amount of the muscles would gradually decrease. Also, if it takes a long time from the time when the muscles has entered the "superrecover" state to the time when the next training interval of time starts, the total amount of the muscles also gradually decreases and sufficient advantageous effect of the training can not be produced. In order to achieve efficient training, it is important to appropriately grasp whether the muscles are in the "superrecovery" state.

Transceiver 340 functions to make wireless communication with myoelectric potential measuring device 10. Transceiver 340 demodulates a signal received from measuring device 10 via the antenna (not shown) and delivers a resulting signal to CPU 320, which modulates and amplifies the signal, and then sends it to controller 30 via the antenna.

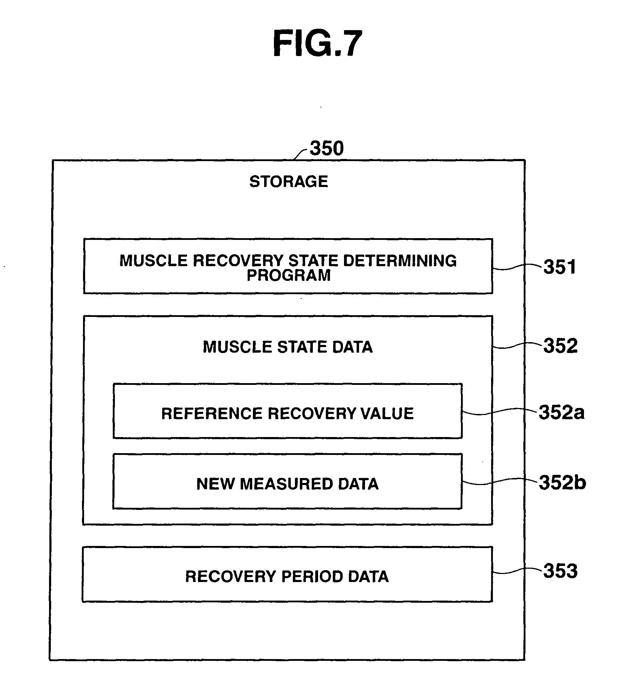

Storage 350 is implemented by IC memories such as a ROM and/or RAM, information storage media such as a hard disk and a reader for the memory or information storage media. CPU 320 has stored a program that controls controller 30. FIG. 7 shows a specified configuration of storage 350. In order to implement the present embodiment, storage 350 has stored a muscle recovery determining program 351 that executes a corresponding process. Storage 350 also has stored a reference recovery value 352a and new measured data 352b as muscular state data 352, and recovery period data 353 indicative of a time taken from the end of the training to the "superrecovery" state.

Muscular state data 352 is not limited to reference recovery value 352a and new measured data 352b. For example, when myoelectric potential can also be measured in general cyclic training, the number of pedalings, muscular strength continuation time (Tv), myoelectric potential generation interval (Tsyc), averaged rectified value (ARV) and median power frequency (MPF) as results of analysis of the training data received from myoelectric potential measuring device 10 are stored in correspondence to the number of pedalings allocated in order of reception.

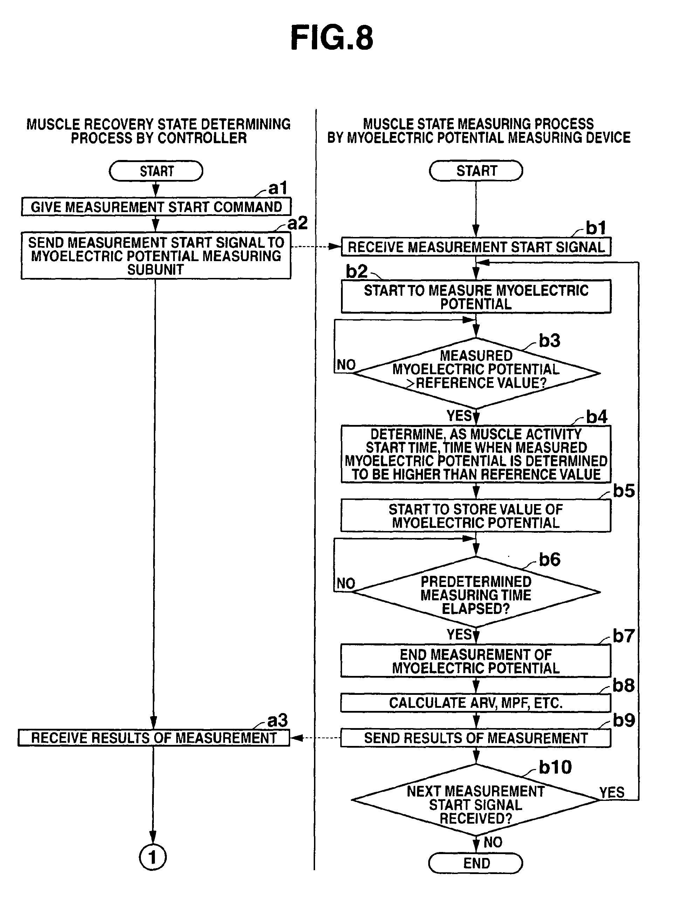

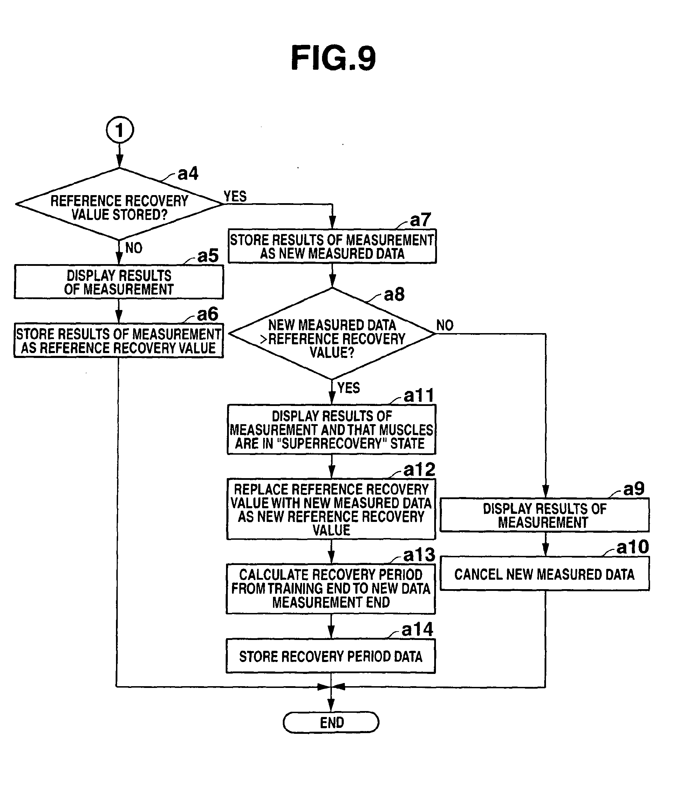

Referring to FIGS. 8 and 9, the processings which CPUs 120 and 320 of myoelectric potential measuring device 10 and controller 30 perform by reading muscular state analysis program 141 and muscle recovery state determining program 351, respectively, will be described. Controller 30 terminates the processing when receiving a measurement termination command from input unit 310 or based on the program when a predetermined time has elapsed.

Before physical training, the user takes isometric exercise and a myoelectric potential occurring in the isometric exercise will be measured. More particularly, when a measurement start command is given at input unit 310 of controller 30 (step a1), CPU 320 sends a measurement start signal to myoelectric potential measuring device 10 (step a2). When the start and end points of a measurement period of time are set at input unit 310, CPU 320 sends signals indicative of the start and ends points of the measurement period of time along with the measurement start signal to myoelectric potential measuring device 10. In the following, it is assumed that the start and end points of the measurement period of time are not specially set at input unit 310, and that the measurement starts at a time when the myoelectric potential exceeds a threshold level set as a default value and continues for 30 seconds indicative of the measurement period of time.

In response to the measurement start signal from controller 30, CPU 120 of device 10 performs the muscular state analysis process. That is, on receiving the measurement start signal (step b1), CPU 120 starts to measure a produced myoelectric potential continuously in measurement subunit 110 (step b2).

Then, CPU 120 compares the measured myoelectric potential with the threshold. If the measured myoelectric potential is below the threshold (NO in step b3), CPU 120 iterates the comparison in step b3. When the measured myoelectric potential exceeds the threshold (YES in step b3), CPU 120 determines this time as the muscular activity start time (ta) 143 (step b4).

In this case, CPU 120 starts to record the myoelectric potential at the determined muscular activity start time (ta) 143, makes measurement for a predetermined period of time (in this embodiment, 30 seconds), and stores obtained data as myoelectric potential data 142 in storage 140 (step b5). CPU 120 also determines whether or not the 30-second measurement period of time has elapsed from the muscular activity start time (ta) (step b6). If not (NO in step b6), CPU 120 further continues to make measurement while determining whether or not the elapsed time has exceeded 30 seconds at which time CPU 120 terminates the measurement (step b7).

Then, CPU 120 calculates an ARV, RMS, integrated value, MPF, median frequency and correlation coefficient based on the myoelectric potential data produced in the measurement period of time (step b8) and then stores them in storage 140. Then, CPU 120 sends these data as results of the analysis to controller 30 (step b9).

Then, CPU 120 determines whether a next measurement start signal is received from controller 30 (step b10). If not (NO in step b10), CPU 120 terminates the processing. If so (YES in step b10), CPU 120 returns to step b2 to iterate the process for measuring and analyzing the muscular state.

If CPU 320 of controller 30 receives results of the analysis (step a3), it determines whether a reference recovery value is stored as muscular state data 352 in storage 350 (step a4). If not (NO in step a4), CPU 320 displays the received results of the analysi+s on display 330 (step a5), and then stores them as a reference recovery value 352a of the muscular state data 352 in storage 350 (step a6).

When a myoelectric potential produced in the isometric exercise across the user's muscles which have taken sufficient rest after the training is being measured and analyzed, results of the analysis of the myoelectric potential information are stored as reference recovery value 352a in storage 350 (YES in step a4). In this case, CPU 320 stores the results of the analysis as new measured data in storage 350 (step a7). Then, CPU 320 determines whether the value of the new measured data is above the reference recovery value, or whether a value obtained by dividing the new measured data by the reference recovery value is above 1 unity (step a8). If not (NO in step a8), CPU 320 displays the results of the analysis (or new measured data) on display 330 (step a9) and then nullifies the results of the analysis (or new measure data) (step a10).

If the new measured data is above the reference recovery value (YES in step a8), CPU 320 displays on display 330 the results of the analysis (or new measured data) and a message that the muscles are in the "superrecovery" state (step a11). CPU 320 also replaces the reference recovery value 352a stored in storage 350 with the results of the analysis (or new measure data) 352b (step a12). Then, CPU 320 calculates a (recovery) period of time from the end point of the previous training to the time when the new measured data was measured (step a13) and then stores calculated recovery period of time data 353 in storage 350 (step a14). The time when the training is terminated may be one when measuring device 10 terminates its measurement or may be fixed by giving a termination command at input unit 310. Then, CPU 320 stores data on the training end point in storage 350.

When next training ends after the recovery period of time data is acquired, CPU 320 times an elapsed time from the time when the training has ended. Then, when the elapsed time exceeds the recovery period of time whose data is stored in storage 350, CPU 320 displays this fact on display 330 such that the user can know it. Before the recovery period of time data is acquired, CPU 320 may not display the message. Alternatively, arrangement may be such that data on a time when the user's muscles are generally presumed to have entered the "superrecovery" state is beforehand stored in storage 350 and when that time comes, CPU 320 informs the user of that fact. Thus, the user can easily know a time when the user's myoelectric potential based on the isometric exercise should be measured again after a non-training interval of time following the training.

When storing the result of the analysis as the reference recovery values in storage 350 (step a6), when canceling the new measured data (step a10), or when storing the recovery period of time data in storage 350 (step a14), CPU 320 at all times determines whether next results of analysis are received from measuring device 10 (step a15). If so (YES in step a15), CPU 320 returns to step a4 to iterate the processing concerned. If not (NO in step a15), CPU 320 terminates the muscular recovery state determining process.

As described above, according to the present embodiment, it is determined based on the myoelectric potential measured by measuring device 10 worn on the user's body before or after the training whether the user's muscles are sufficiently recovered to the "superrecovery" state suitable for next training. Thus, user can accurately grasp a time when the next training should be taken, thereby allowing efficient training to be taken.

Myoelectric potential measuring device 10 may be composed so as to measure various living body information such as myoelectric potentials, blood pressures and pulses on the user's body when he or she is taking training.

While in the embodiment measurement of a myoelectric potential produced across the biceps of the user's upper arm with measuring device 10 worn on the biceps has been illustrated, the part of the user's body on which measuring device 10 is worn for measuring purpose is not limited to the above particular example. It may be any of other muscles of the user's arms and legs such as a triceps thigh rectus, biceps muscles of thigh and soleus muscles or further other muscles of the user where the myoelectric potential is desired to be measured, thereby allowing the fatigue/recovery state of the muscles to be known.

While in the embodiment the number of myoelectric potential measuring devices 10 is especially referred to, two or more such devices may be worn on appropriate parts of the living body for measuring purposes, as shown in FIG. 2.

Controller 30 may be such that muscular state data 352 and recovery period of time data 353 which are illustrated as stored in storage 350 can be instead stored on a portable information storage medium such as a memory card set in the controller so as to be usable on an external device, for example, a personal computer. Alternatively, a functional element which sends the respective data to external devices may be provided in controller 30.

In order to implement transceivers 130 and 340 in an inexpensive manner, arrangement may be such that the measurement start and end points and hence the measurement period of time are set in myoelectric potential measuring device 10. In this case, for transmitting and receiving functions, measuring device 10 may include only the transmitting function whereas controller 30 includes only the receiving function. It is noted in this case that measuring device 10 requires an input unit.

While in the embodiment myoelectric potential measuring device 10 and controller 30 are illustrated as separate devices of the living body information measuring apparatus, the measuring apparatus may include, in its single body, the myoelectric potential measuring submit, storage that stores the myoelectric potential information acquired by the measuring submit, and a determining unit that determines an extent of recovery of the muscles from the fatigue based on results of the measurement.

While in the embodiment display 330 is illustrated as the unit that informs the user that the predetermined recovery time has elapsed since the end of the training, the informing unit is not limited to that illustrated. For example, it may include an acoustic device that generates an alarm, a lamp that is lighted to inform the user that the predetermined recovery time has elapsed.

The structures of the embodiments according to the present invention and advantages produced thereby are summarized as follows:
1) In one embodiment, a living body information measuring apparatus comprises:
   a measuring unit (10 in FIG. 2) for measuring a myoelectric potential produced across a predetermined muscle of a living body in isometric exercise;
   storage (350 in FIG. 2) for storing as a reference recovery value an item of myoelectric potential information involving the myoelectric potential measured by the measuring unit before physical training; and
   a determining unit (320 in FIG. 2; step a8 in FIG. 9) for comparing the reference recovery value stored in the storage with the value of the item of myoelectric potential information involving a myoelectric potential across the predetermined muscle measured by the measuring unit after the training to determine an extent of recovery of the muscle.
   Thus, according to this embodiment, although the recovering speed of a user's muscle is different from that of another user and varies depending on a part of the former user's body that the muscle involves, an extent of recovery of his or her muscle from fatigue can be determined by comparing the reference recovery value acquired before the training with a value of the item of myoelectric potential information obtained after the training. Thus, the time when the muscle has entered the "superrecovery" state is grasped without relying on the user's sensations, thereby allowing appropriate training to be taken.
2) In one embodiment, in the apparatus of item 1 the item of myoelectric potential information comprises at least one of an averaged rectified value, a root mean square value and an integrated value as (FIG. 4A), estimated indexes, for the amplitude of a waveform involving the measured myoelectric potential within a predetermined measurement period of time.
   Thus, according to this embodiment, the time when the muscle has entered the superrecovery state is grasped based on such item of myoelectric potential inforamtion, thereby allowing appropriate physical training to be done.
3) In one embodiment, in the apparatus of item 1) the item of myoelectric potential information comprises at least one of a mean power frequency and a median frequency (FIG. 4B) as estimated indexes for the frequency of a waveform involving the measured myoelectric potential within a predetermined measurement period of time.
   Thus, according to this embodiment, advantages similar to those produced by the apparatus of item 2) are produced.
4) In one embodiment, in the apparatus of item 1) the item of myoelectric potential information comprises a correlation coefficient between the evaluated indexes for the amplitude and frequency in the predetermined measurement period of time (FIG. 4C).
   Thus, also according to this embodiment, advantages similar to those produced by the apparatus of item 2) are produced.
5) In the apparatus of item 1), when the value of the item of myoelectric potential information obtained after the training is larger than the reference recovery value, the determining unit (320 in FIG. 2) determines that the predetermined muscle has been recovered.
   Thus, according to this embodiment, a time when the muscle has entered the "superrecovery" state can be grasped, thereby allowing appropriate training to be taken.
6) In one embodiment, in the apparatus of item 2, the predetermined measurement period of interval starts a selected time after the measuring unit has started to measure the myoelectric potential (FIGS. 6A, 6B).
   The myoelectric potential measured when the muscle is fatigued starts to change some time after the start of the measurement although the time when the myoelectric potential starts to change varies from user to user. Thus, according to the last-mentioned embodiment, the measurement period of time is arranged so as to start the selected time after the start of the measurement, thereby allowing efficient measurement of the myoelectric potential to be acheived.
7) In one embodiment, the apparatus of item 2) further comprises a setting unit (320 in FIG. 2) that sets the start and end points of the predetermined measurement period of time.
   Thus, according to this embodiment, the user can at will set the start and end points of the measurement period of time at the setting unit, for example, depending on a characteristic of advent of the user's fatigue, thereby allowing the myoelectric potential to be measured efficiently.
8) In one embodiment, in the apparatus of item 1) the storage stores the item of measured myoelectric potential information along with a date when the associated myoelectric potential was measured.
   Thus, according to this embodiment, a plurality of items of the myoelectric potential information can be stored in a time series manner along with the corresponding dates when the respective myoelectric potentials were measured.
9) In one embodiment, in the apparatus of item 1) the storage (350 in FIG. 7) stores data on a recovery period of time between the end of the training and a time when the determining unit determines that the value of the item of myoelectric potential information after the training has exceeded the reference recovery value, and further comprising :
   an informing unit (330 in FIG. 2) that when a time corresponding to the recovery period of time has elapsed from the end of next training, informs of this fact.
   Thus, according to this embodiment, the user can appropriately determine when measurement should be made to confirm the recovered state of the muscle.
10) In one embodiment, a living body information measuring system comprises:
   a measuring device (10 in FIG. 2) for measuring information on the respective parts of a living body; and
   a controller (30) provided separately from the measuring device, and wherein:
      the measuring device comprises:
         a measuring subunit (110) for measuring myoelectric potentials produced across a predetermined muscle of a living body in isometric exercise before and after physical training; and
         a sending unit (130) for sending the controller a plurality of items of myoelectric potential information involving the myoelectric potentials measured by the measuring subunit before and after the physical training; and
         the controller (30) comprises:
            a receiving unit (340) for receiving the plurality of items of myoelectric potential information from the measuring subunit;
            storage (350) for storing as a reference recovery value an item of myoelectric potential information involving the myoelectric potential measured by the measuring subunit before the physical training; and
            a determining unit (320) for comparing the reference recovery value stored in the storage with a value of the item of myoelectric potential information involving the myoelectric potential measured after the training by the measuring subunit to determine an extent of recovery of the predetermined muscle.

Thus, according to this embodiment, advantages similar to those produced by the apparatus of item 1) are produced as well.

Various modifications and changes may be made thereunto without departing from the broad spirit and scope of this invention. The above-described embodiments are intended to illustrate the present invention, not to limit the scope of the present invention. The scope of the present invention is shown by the attached claims rather than the embodiments. Various modifications made within the meaning of an equivalent of the claims of the invention and within the claims are to be regarded to be in the scope of the present invention.

This application is based on Japanese Patent Application No. 2006-144388 filed on May 24, 2006 and including specification, claims, drawings and summary. The disclosure of the above Japanese patent application is incorporated herein by reference in its entirety.

## Claims

1. A living body information measuring apparatus comprising:
a measuring unit (10) for measuring a myoelectric potential produced across a predetermined muscle of a living body in isometric exercise;
storage (350) for storing as a reference recovery value a value of an item of myoelectric potential information involving the myoelectric potential measured by the measuring unit before physical training; and
a determining unit (320) for comparing the reference recovery value stored in the storage with a value of an item of myoelectric potential information involving a myoelectric potential across the predetermined muscle measured by the measuring unit after the training to determine an extent of recovery of the muscle.

2. The apparatus of claim 1, wherein the item of myoelectric potential information comprises at least one of an averaged rectified value, a root mean square value and an integrated value as estimated indexes for the amplitude of a waveform involving the measured myoelectric potential within a predetermined measurement period of time.

3. The apparatus of claim 1, wherein the item of myoelectric potential information comprises at least one of a mean power frequency and a median frequency as estimated indexes for the frequency of a waveform involving the measured myoelectric potential within a predetermined measurement period of time.

4. The apparatus of claim 1, wherein the item of myoelectric potential information comprises a correlation coefficient between the evaluated indexes for the amplitude and frequency in the predetermined measurement period of time.

5. The apparatus of claim 1, wherein when the value of the item of myoelectric potential information involving the myoelectric potential measured after the training is larger than the reference recovery value, the determining unit (320) determines that the predetermined muscle has been recovered.

6. The apparatus of claim 2, wherein the predetermined measurement period of interval starts a selected time after the measuring unit has started to measure the myoelectric potential.

7. The apparatus of claim 2, further comprising a setting unit (320)that sets the start and end points of the predetermined measurement period of time.

8. The apparatus of claim 1, wherein the storage stores the item of myoelectric potential information along with a date when the associated myoelectric potential was measured.

9. The apparatus of claim 1, wherein the storage(350) stores data on a recovery period of time between the end of the training and a time when the determining unit determines that the value of the item of myoelectric potential information involving the myoelectric potential measured after the training has exceeded the reference recovery value, and further comprising:
an informing unit (330)that when a time corresponding to the recovery period of time has elapsed from the end of next training, informs of this fact.

10. A living body information measuring system comprising:
a measuring device (10)for measuring information on the respective parts of a living body; and
a controller (30)provided separately from the measuring device, and wherein:
the measuring device comprises:
a measuring subunit (110)for measuring myoelectric potentials produced across a predetermined muscle of a living body in isometric exercise before and after physical training; and
a sending unit (130)for sending the controller a plurality of items of myoelectric potential information involving the myoelectric potentials measured by the measuring subunit; and
the controller comprises:
a receiving unit (340)for receiving the plurality of items of myoelectric potential information involving the measured myoelectric potentials from the measuring subunit;
storage (350)for storing as a reference recovery value the item of myoelectric potential information involving the myoelectric potential measured by the measuring subunit before the training among the plurality of items of myoelectric potential inforamtion received from the measuring subunit; and
a determining unit (320)for comparing the reference recovery value stored in the storage with a value of the item of myoelectric potential information involving the myoelectric potential measured after the training by the measuring subunit to determine an extent of recovery of the predetermined muscle.
